# EUROPEAN PATENT APPLICATION

(11) **EP 1 374 831 A1**
(43) Date of publication of application: **02.01.2004**
(21) Application number: 02702766.3
(22) Date of filing: 06.03.2002
(51) Int. Cl.: A61K 7/00, A61K 7/50, A61K 7/075, A61K 7/06, A61K 31/223, A61P 17/16, A61P 17/00

(54) **COMSETICS OR EXTERNAL PREPARAIOTNS FOR SKIN**

(30) Priority: 13.03.2001 JP 2001070322
(71) Applicant: Ajinomoto Co., Inc., Tokyo 104-8315 (JP)
(72) Inventor: IWASAKI, Keiji, c/o Amino Science Laboratories, Kawasaki-shi, Kanagawa 210-8681 (JP); KITAZAWA, Manabu, c/o Amino Science Laboratories, Kawasaki-shi, Kanagawa 210-8681 (JP); SAKAMOTO, Kazutami, c/o Amino Science Laboratories, Kawasaki-shi, Kanagawa 210-8681 (JP)
(74) Representative: Nicholls, Kathryn Margaret
(86) International application number: PCT/JP2002/002082
(87) International publication number: WO 2002/072040

(57) **Abstract**

According to the present invention, there is provided cosmetics or external preparations for skin which comprises, as the effective ingredient, a chemical peeling agent, a bactericide, an anionic surfactant or a cationic surfactant component in combination with a compound capable of mitigating the irritation, inflammation, etc. of the skin caused by these components.

The present invention is skin cosmetics, external preparations for skin or hair cosmetics which are characterized by containing the following components (A) and (B) or it is skin cosmetics or external preparations for skin which are characterized by containing the following components (C) and (D)
Components (A) and (C): one or more members selected from a cystine derivative and salt thereof
Component (B): one or more of members selected from a chemical peeling agent, a bactericide and an anionic surfactant
Component (C): a cationic surfactant

## Description

### Technical Field

The present invention relates to cosmetics or external preparations for skin which comprises, as the effective ingredient, a chemical peeling agent, a bactericide, an anionic surfactant or a cationic surfactant and a compound capable of mitigating irritation, inflammation, etc. on the skin caused by these components.

### Background Art

Among the raw materials for use in cosmetics, external preparations for skin or the like, there are not few ones which bring an irritation, an inflammation and so on to the skin. For example, chemical peeling agents such as lactic acid, glycolic acid and so on have been used for the purpose of removing fine wrinkles or the like owing to their keratin turnover-accelerating effect (chemical peeling effect) but they bring irritation to the skin at a certain concentration and pH so that their use is limited. Although a bactericide such as benzalkonium chloride or the like, an anionic surfactant such as sodium lauryl sulfate or the like, or a cationic surfactant such as stearyl trimethyl ammonium chloride or the like has been widely used in a variety of cosmetics and external preparations for skin duing to its bactericidal effect, detergent effect, or disinfectant and detergent effect, similarly each of them brings the irritation, inflammation and so on to the skin so that the use is limited. It is known that regarding inflammatory citokines such as IL-1 α, TNF- α , etc., they are closely related to the irritation, inflammation or the like to be brought to the skin as its cause (e.g. "Oxidative Stress in dermatology", Marcel Dekker, Inc., pp. 187-205, 1993). Expression of a gene coding these proteins is mainly controlled at the level of genetic transcription whereas regarding inflammatory citokines, their expression is controlled by a transcriptional regulatory factor such as NF- κ B and AP-1 (e.g. "Active oxygen and Signal transmission", Kodansha Scientific, pp. 37-46, 1996).

As a compound which inhibits the activation of NF- κ B, sulfur-containing antioxidants such as N-acetyl-L-cysteine and pyrrolidine dithiocarbamate are known (e.g. "Active oxygen and Signal transmission", Kodansha Scientific, pp. 37-46, 1996). However, when these sulfur-containing antioxidants were used as cosmetics or external preparations for skin, they have foreign odor to give the users unfavorable feeling. N, N'-Diacetyl-L-cystine dimethyl ester is known to suppress the activation of both NF- κ B and AP-1 (e.g. WO/2000-21925) and to have no unfavorable foreign odor. Regarding N, N'-diacetyl-L-cystine dimethyl ester, however, it has not been known that by using it in combination with hydroxy acid, a bactericide, an anionic surfactant or a cationic surfactant, the irritation and so on caused by these components is mitigated. In addition to sulfur-containing antioxidants, there are reported that the activation of AP-1 is inhibited by retinoic acid (e.g. "Nature", vol. 379, pp. 335-339, 1996), that the activation of NF- κ B is inhibited by steroidal anti-inflammatory drugs or non-steroidal anti-inflammatory drugs (e.g. "Bio Essays", vol. 18, pp.371-378 1996), etc. However, retinoic acid and steroidal anti-inflammatory drugs have side effects such as detachment of skin and steroid-induced skin diseases, respectively so that their use is limited. Non-steroidal anti-inflammatory drugs do not cause systemic side effects which are observed upon use of a steroidal anti-inflammatory drug but there is room for improvement in their local side effects. In addition, their effects for suppressing the activation of inflammatory factors are insufficient.

### Disclosure of the Invention

An object of the present invention is to provide cosmetics or external preparations for skin which comprises, as the effective ingredient, a chemical peeling agent, a bactericide, an anionic surfactant or a cationic surfactant and a compound capable of mitigating irritation, inflammation, etc. of the skin caused by these components.

As a result of having ardently studied to achieve the above object, the present inventors have found that by using a cystine derivative which may be represented by the following general formula ( I ) in combination with a chemical peeling agent, a bactericide, an anionic surfactant or a cationic surfactant, the irritation, inflammation, etc. of the skin caused by the chemical peeling agent, the bactericide, the anionic surfactant or the cationic surfactant may be mitigated, and has completed the present invention.

That is, the present invention relaters to skin cosmetics, external preparations for the skin or hair cosmetics which are characterized by containing the following components (A) and (B), and to skin cosmetics or external preparations for the skin which are characterized by containing the following components (C) and (D):
Components (A) and (C): one or more of members selected from a cystine derivative which may be represented by the following general formula (I) and salt thereof
Components (B): one or more of members selected from a chemical peeling agent, a bactericide and an anionic surfactant
Components (D): a cationic surfactant 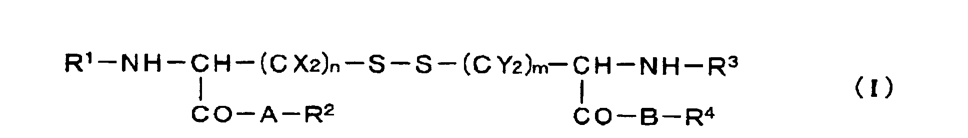
wherein R¹ and R³ represent a hydrogen atom, an aminocarbonyl group, an alkyl group having 1-22 carbon atoms, an acyl group having 2-22 carbon atoms, a hydroxyalkyl group having 1-22 carbon atoms or a 3-alkoxy-2-hydroxypropyl group whose alkoxy group has 1-22 carbon atoms, two X and two Y represent each independently a hydrogen or an alkyl group having 1-6 carbon atoms, and n and m represent each independently an integer of 0-5. A and B represent independently each -O- or -NH-, R² and R⁴ represent a hydrogen atom, an alkyl group having 1-22 carbon atoms, a hydroxyalkyl group having 1-22 carbon atoms or a 3-alkoxy-2-hydroxypropyl group whose alkoxy group has 1-22 carbon atoms.

The followings illustrate the present invention in detail.

In the cystine derivative represented by the above-described general formula ( I ) and salts thereof for use in the present invention, as the examples of R¹ and R³ there may be taken a hydrogen atom, an aminocarbonyl, acetyl, propionyl, isopropionyl, n-butyryl, isobutyryl, sec-butyryl, tert-butyryl, n-valeryl, sec-valeryl, tert-valeryl, isovaleryl, n-hexanoyl, cyclohexanoyl, n-heptanoyl, n-octanoyl, 2-ethylhexanoyl, nonanoyl, isononanoyl,, decanoyl, isodecanoyl, undecanoyl, lauroyl, tridecanoyl, isotridecanoyl, myristoyl, palmitoyl, isopalmitoyl, stearoyl,, isostearoyl, oleoyl, docosanoyl, methyl, ethyl, propyl, isopropyl, n-butyl, isobutyl, sec-butyl, tert-butyl, n-amyl, sec-amyl, tert-amyl, isoamyl, n-hexyl, cyclohexyl, n-heptyl, n-octyl, 2-ethylhexyl, nonyl, isononyl, decyl, isodecyl, undecyl, lauryl, tridecyl, isotridecyl, myristyl, cetyl, isocetyl, stearyl, isostearyl, oleyl, behenyl, 2-hydroxyethyl, 2-hydroxypropyl, 2-hydroxyisopropyl, 2-hydroxy-n-butyl, 2-hydroxyisobutyl, 2-hydroxy-sec-butyl, 2-hydroxy-tert-butyl, 2-hydroxy-n-amyl, 2-hydroxy-sec-amyl, 2-hydroxy-tert-amyl, 2-hydroxyisoamyl, 2-hydroxy-n-hexyl, 2-hydroxycyclohexyl, 2-hydroxy-n-heptyl, 2-hydroxy-n-octyl, 2-hydroxy-2-ethylhexyl, 2-hydroxynonyl, 2-hydroxyisononyl, 2-hydroxydecyl, 2-hydroxyisodecyl, 2-hydroxyundecyl, 2-hydroxyrauryl, 2-hydroxytridecyl, 2-hydroxyisotridecyl, 2-hydroxyimyristyl, 2-hydroxycetyl, 2-hydroxyisocetyl, 2-hydroxystearyl, 2-hydroxyisostearyl, 2-hydroxyoleyl, 2-hydroxybehenyl, 3-methoxy-2-hydroxypropyl, 3-ethoxy-2-hydroxypropyl, 3-propioxy-2-hydroxypropyl, 3-isopropioxy-2-hydroxypropyl, 3-n-buthoxy-2-hydroxypropyl, 3-isobuthoxy-2-hydroxypropyl, 3-sec-buthoxy-2-hydroxypropyl, 3-tert-buthoxy-2-hydroxypropyl, 3-n-amyloxy-2-hydroxypropyl, 3-sec-amyloxy-2-hydroxypropyl, 3-tert-amyloxy-2-hydroxypropyl, 3-isoamyloxy-2-hydroxypropyl, 3-n-hexyloxy-2-hydroxypropyl, 3-cyclohexyloxy-2-hydroxypropyl, 3-n-heptyloxy-2-hydroxypropyl, 3-n-octyloxy-2-hydroxypropyl, 3-(2-ethylhexyl)oxy-2-hydroxypropyl, 3-nonyloxy-2-hydroxypropyl, 3-isononyloxy-2-hydroxypropyl, 3-decyloxy-2-hydroxypropyl, 3-isodecyloxy-2-hydroxypropyl, 3-undecyloxy-2-hydroxypropyl, 3-lauryloxy-2-hydroxypropyl, 3-tridecyloxy-2-hydroxypropyl, 3-isotridecyloxy-2-hydroxypropyl, 3-myristyloxy-2-hydroxypropyl, 3-cetyloxy-2-hydroxypropyl, 3-isocetyloxy-2-hydroxypropyl, 3-stearyloxy-2-hydroxypropyl, 3-isostearyloxy-2-hydroxypropyl, 3-oleoyloxy-2-hydroxypropyl, 3-behenyloxy-2-hydroxypropyl and the like groups.

As examples of R² and R⁴, there may be taken a hydrogen atom, methyl, ethyl, propyl, isopropyl, n-butyl, isobutyl, sec-butyl, tert-butyl, n-amyl, sec-amyl, tert-amyl, isoamyl, n-hexyl, cyclohexyl, n-heptyl, n-octyl, 2-ethylhexyl, nonyl, isononyl, decyl, isodecyl, undecyl, lauryl, tridecyl, isotridecyl, myristyl, cetyl, isocetyl, stearyl, isostearyl, oleyl, behenyl, 2-hydroxyethyl, 2-hydroxypropyl, 2-hydroxyisopropyl, 2-hydroxy-n-butyl, 2-hydroxyisobutyl, 2-hydroxy-sec-butyl, 2-hydroxy-tert-butyl, 2-hydroxy-n-amyl, 2-hydroxy-sec-amyl, 2-hydroxy-tert-amyl, 2-hydroxyisoamyl, 2-hydroxy-n-hexyl, 2-hydroxycyclohexyl, 2-hydroxy-n-heptyl, 2-hydroxy-n-octyl, 2-hydroxy-2-ethylhexyl, 2-hydroxynonyl, 2-hydroxyisononyl, 2-hydroxydecyl, 2-hydroxyisodecyl, 2-hydroxyundecyl, 2-hydroxyrauryl, 2-hydroxytridecyl, 2-hydroxyisotridecyl, 2-hydroxyimyristyl, 2-hydroxycetyl, 2-hydroxyisocetyl, 2-hydroxystearyl, 2-hydroxyisostearyl, 2-hydroxyoleyl, 2-hydroxybehenyl, 3-methoxy-2-hydroxypropyl, 3-ethoxy-2-hydroxypropyl, 3-propioxy-2-hydroxypropyl, 3-isopropioxy-2-hydroxypropyl, 3-n-buthoxy-2-hydroxypropyl, 3-isobuthoxy-2-hydroxypropyl, 3-sec-buthoxy-2-hydroxypropyl, 3-tert-buthoxy-2-hydroxypropyl, 3-n-amyloxy-2-hydroxypropyl, 3-sec-amyloxy-2-hydroxypropyl, 3-tert-amyloxy-2-hydroxypropyl, 3-isoamyloxy-2-hydroxypropyl, 3-n-hexyloxy-2-hydroxypropyl, 3-cyclohexyloxy-2-hydroxypropyl, 3-n-heptyloxy-2-hydroxypropyl, 3-n-octyloxy-2-hydroxypropyl, 3-(2-ethylhexyl)oxy-2-hydroxypropyl, 3-nonyloxy-2-hydroxypropyl, 3-isononyloxy-2-hydroxypropyl, 3-decyloxy-2-hydroxypropyl, 3-isodecyloxy-2-hydroxypropyl, 3-undecyloxy-2-hydroxypropyl, 3-lauryloxy-2-hydroxypropyl, 3-tridecyloxy-2-hydroxypropyl, 3-isotridecyloxy-2-hydroxypropyl, 3-myristyloxy-2-hydroxypropyl, 3-cetyloxy-2-hydroxypropyl, 3-isocetyloxy-2-hydroxypropyl, 3-stearyloxy-2-hydroxypropyl, 3-isostearyloxy-2-hydroxypropyl, 3-oleoyloxy-2-hydroxypropyl, 3-behenyloxy-2-hydroxypropyl and the like groups.

The cystine derivative represented by the above-described general formula ( I ) may be either in the optically active form or racemic form, but L-and DL-forms are preferred. Also, as examples of the salt of the compound represented by the above-described general formula ( l ), there may be taken salts of an inorganic acid such as hydrochloride, hydrobromide, hydroiodide, nitrate, sulfate, phosphate and the like; salts of organic acids such as methanesulphonate, ethanesulphonate, benzenesulphonate, p-toluenesulphonate, 1-camphorsulphonate, acetate, lactate, citrate, tartrate, succinate, maleate, fumarate, gluconate, glycolate, saccharate, benzoate, a fatty acid salt, malate, pyrroglutamate and the like: salts of an acidic amino acid such as aspartic acid, glutamic acid and the like. These salts may be used singly or in combination with one or more kinds of them. Furthermore, one or more of salts may be used in combination with a free form.

As a process for preparing the cystine derivative represented by the above-described general formula ( l ), for example, there may be taken a process wherein L- or DL-cystine is reacted with an acid anhydride, an acid chloride, a halogenated alkyl, an epoxyalkane or an alkyl glycidyl ether thereby the amino group is acylated, alkylated, hydroxyalkylated or 3-alkoxy-2-hydroxypropylated and thereafter the resulting product is subjected to dehydration-condensation with an alkylamine or an alcohol to lead into its amide or ester form. Also, for example there may be taken one wherein L- or DL-cystine diester is reacted with an acid anhydride, an acid chloride, a halogenated alkyl, an epoxyalkane or an alkyl glycidyl ether thereby an amino group is acylated, alkylated, hydroxyalkylated or 3-alkoxy-2-hydroxypropylated. Also, for example there may be taken one wherein L- or DL-cystine diamide is reacted with an acid anhydride, an acid chloride, a halogenated alkyl, an epoxyalkane or an alkyl glycidyl ether thereby an amino group is acylated, alkylated, hydroxyalkylated or 3-alkoxy-2-hydroxypropylated.

As the chemical peeling agent of Component (B), there may be taken a hydroxy acid, its derivative and salts thereof. As the hydroxy acid, there may be taken glycolic acid, lactic acid, methyl lactate, 3-phenyllactic acid, 3-chlorolactic acid, 1-hydroxy-1-cyclopropanecarboxylic acid, 2-hydroxybutyric acid, 3-hydroxybutyric acid, 4-hydroxybutyric acid, 2-hydroxypentanoic acid, 2-hydroxycaproic acid, 2-hydroxyheptanoic acid, 2-hydroxyoctanoic acid, 2-hydroxynonanoic acid, 2-hydroxydecanoic acid, 10-hydroxydecanoic acid, 2-hydroxyundecanoic acid, 2-hydroxylauric acid, 12-hydroxylauric acid, 2-hydroxymyristic acid, 3-hydroxymyristic acid, 2-hydroxypalmitic acid, 16-hydroxypalmitic acid, 2-hydroxystearic acid, 2-hydroxyeicosanic acid, 2-hydroxytetraeicosanic acid, 2-hydroxytetraeicosenic acid, mandelic acid, 2,2-diphenyl-2-hydroxyacetic acid, 2-phenyl-2-methyl-2-hydroxyacetic acid, 2-(4'-hydroxyphenyl)-2-hydroxyacetic acid, 2-(4'-chlorophenyl)-2-hydroxyacetic acid, 2-(3'- hydroxy-4'-methoxyphenyl)-2-hydroxyacetic acid, 3-(2'-hydroxyphenyl)-2-hydroxypropionic acid, 3-(4'-hydroxyphenyl)-2-hydroxypropionic acid, 3-(3', 4'-dihydroxyphenyl)-2-hydroxyacetic acid, glyceric acid, 2,3,4-trihydroxybuttric acid (erythronic acid , threonic acid, etc.), 2, 3, 4, 5-tetrahydroxypentanoic acid (ribonic acid, arabinonic acid, xylonic acid, lyxononic acid, etc.), 2, 3, 4, 5, 6-pentahydroxyhexanoic acid (allonoc acid, altronic acid, gluconic acid, mannonic acid, gulonic acid, idonic acid, galactonic acid, talonic acid, etc.), 2, 3, 4, 5, 6, 7-hexahydroxyheptanoic acid (isomer, glucoheptonic acid, galactoheptonic acid, etc.), tartronic acid, malic acid, tartaric acid, citric acid, isocitric acid, 2, 3, 4, 5-tetrahydroxyhexane-1,6-dioic acid (glucaric acid, mannaric acid, saccharic acid, mucic acid, etc.) quinic acid, salicylic acid, tropic acid, trethochanic acid, citramalic acid, agaricic acid, aleuritic acid, pantoic acid, lactobionic acid, hexuronic acid, etc. As other chemical peeling agent than the hydroxy acid, there can be taken lactones such as gluconolactone, galactonolactone, glucuronolactone, galacturonolactone, glonolactone, ribonic- γ -lactone, aldonic acid lactone, pantoactone, glucoheptonic-γ-lactone, mannonic-γ-lactone, galactoheptonic lactone and the like; keto acids such as glyoxylic acid, methyl 2-ketoglyoxylate, pyruvic acid, methyl pyruvate, ethyl pyruvate, propyl pyruvate, benzoylformic acid, methyl benzoylformate, ethyl benzoylformate, phenylpyruvic acid, methyl phenylpyruvate, ethyl phenylpyruvate, 2-ketobutyric acid, 2-ketopentanoic acid, 2-ketohexanoic acid, 2-ketoheptanoic acid, 2-ketooctanoic acid, 2-ketododecanoic acid, methyl 2-ketooctanate; trichloroacetic acid, resorcinol, phenol and the like. Furthermore, there can be taken natural products or natural extracts containing the hydroxy acid or other chemical peeling agent than the hydroxy acid such as a honey extract, an apple extract, sugar cane extract, tropical fruit extract, an extract of multifruit berry, etc.

Also, as examples of the bactericide of the above-described Component (B), there can be taken benzalkonium chloride, phenol, chlorophenol, chloro-*m*-cresol, *p*-chloro-*m*-xylenol, isopropyl methyl phenol, resorcine, resorcine acetate, *o*-phenylphenol, phenoxyethanol, thymol, cresol, hinokitiol, thioxolone, benzoic acid, sodium benzoate, salicylic acid, sodium salicylate, dehydroacetic acid and salts thereof, sorbic acid and salts thereof, hexachlorophene, trichlorohydroxydiphenyl ether (triclosan), trichlorocarbanilide, halocarboxylic acid, monoethanolamide undecylenate, alkyl isoquinolium bromide, benzethonium chloride, cetylpyridinium chloride, decamethonium chloride, alkyl aminoethylglycine chloride, stearyl chloride hydroxy ethyl betaine sodium, chlorhexidine gluconate, chlorhexidine hydrochloride, thiram, photosensitive element, zinc pyrithione, lysozyme chloride, chlorobutanol, chlorphenesin, povidone-iodine, acrinol, boric acid, isopropanol, glutaraldehyde, formalin, sodium hypochloride, sodium dichloroisocyanurate, chloramines-T, domiphen bromide, etc.

As examples of the anionic surfactant of the above-described Component (B), there can be taken salts of higher fatty acids such as lauric acid, mtristic acid, palmytic acid, stearic acid, coconut oil fatty acid, hydrogenated tallow oil fatty acid, oleic acid and the like; salts of N-acylamino acids such as N-long chain acylglutamic acid, N-long chain acylaspaltic acid, N-long chain acylglycine, N-long chain acylalanine, N-long chain acylsarcosine, N-long chain acyl- *β* - alanine, N-long chain acyl-N-methyl- *β* -alanine and the like; ether carboxylates such as sodium polyoxyethylene (3E. O.) lauryl ether acetate, polyoxyethylene (3E. O.) lauryl ether acetic acid, sodium polyoxyethylene (3E.O.) tridecyl ether acetate, polyoxyethylene (3E. O.) tridecyl ether acetic acid, sodium polyoxyethylene (4.5E. O.) lauryl ether acetate, sodium polyoxyethylene (6E. O.) tridecyl ether acetate, polyoxyethylene (7E. O.) tridecyl ether acetic acid, sodium polyoxyethylene (3E. O.) stearyl ether acetate, sodium polyoxyethylene (3E. O.) octyl ether acetate, monoethanolamine polyoxyethylene (3E. O.) lauryl ether acetate and the like; alkyl sulfonates such as triethanolamine dodecylbenzenesulfonte, sodium dodecylbenzenesulfonate, sodium α -olefinsulfonate (carbon number of 12) and the like; sulfosuccinates such as sulfosuccinic acid dioctyl ester sodium salt, sulfosuccinic acid lauryl ester disodium salt, sulfosuccinic acid tallow amide disodium salt, polyoxyethylene (1-5E. O.) sulfosuccinic acid lauryl ester disodium salt, polyoxyethylene (3E. O.) sulfosuccinic acid myrisyl ester disodium salt, sulfosuccinic acid polyoxyethylene (5E. O.) lauroyl ethanolamide disodium salt, sulfosuccinic acid polyoxyethylene (2E. O.) monooleylamide disodium salt and the like; N-acylsulfonates such as sodium salt of cocoyl-N-methyltaurine, sodium salt of lauroyl-N-methyltaurine, triethanolamine salt of cocoyl-N-methyltaurine, triethanolamine salt of lauroyl-N-methyltaurine and the like; O-acylsulfonates such as sodium cocoylisethionate, sodium lauroylisethionate, triethanolamine cocoylisethionate, triethanolamine lauroylisethionate and the like; alkyl sulfates such as sodium lauryl sulfate, sodium myristyl sulfate, sodium palmityl sulfate, triethanolamine lauryl sulfate, triethanolamine myristyl sulfate triethanolamine palmityl sulfate and the like; ether sulfates such as triethanolamine polyoxyethylene (2-4E. O.) lauryl ether sulfate, sodium polyoxyethylene (2-4E. O.) myristyl ether sulfate, sodium polyoxyethylene (2-4E. O.) palmityl ether sulfate, triethanolamine polyoxyethylene (2-4E. O.) palmityl ether sulfate and the like; alkyl phosphates such as sodium lauryl phosphate, triethanolamine lauryl phosphate, sodium myristyl phosphate, triethanolamine myristyl phosphate, sodium oleyl phosphate, triethanolamine oleyl phosphate, sodium polyoxyethylene (2-4E. O.) lauryl ether phosphate, triethanolamine polyoxyethylene (2-4E. O.) lauryl ether phosphate, sodium polyoxyethylene (2-4E. O.) myristyl ether phosphate, triethanolamine polyoxyethylene (2-4E. O.) myristyl ether phosphate, sodium polyoxyethylene (2-4E. O.) oleyl ether phosphate, triethanolamine polyoxyethylene (2-4E. O.) oleyl ether phosphate, and the like.

Also, as examples of the cationic surfactant of the above-described Component (D), there can be taken salts of N^{α} -long chain acylarginine lower alkyl ester such as N^{α} -cocoylarginine ethyl ester DL-pyrrolidone carboxylate, N^{α} -cocoylarginine ethyl ester hydrochloride, N^{α} -cocoylarginine ethyl ester lactate, N^{α}-cocoylarginine ethyl ester sucinate, N^{α} -cocoylarginine ethyl ester citrate, N^{α}-cocoyllarginine ethyl ester malate, N^{α}-cocoylarginine ethyl ester phosphate, N^{α} -cocoylarginine ethyl ester glutamate, N^{α}-cocoylarginine ethyl ester aspartate, N^{α}-cocoylarginine ethyl ester salfate, N^{α} -cocoylarginine methyl ester DL-pyrrolidone carboxylate, N^{α} -lauroylarginine ethyl ester DL-pyrrolidone carboxylate, N^{α} -myristoylarginine ethyl ester DL-pyrrolidone carboxylate, N^{α} -palmitoylarginine ethyl ester DL-pyrrolidone carboxylate, N^{α} -stearoylarginine ethyl ester DL-pyrrolidone carboxylate, N^{α} -oleoylarginine ethyl ester DL-pyrrolidone carboxylate and the like; salts of N^{ε}-acyllysine lower alkyl ester such as N^{ε} lauroyllysine ethyl ester DL-pyrrolidone carboxylate, N^{ε} -lauroyllysine ethyl ester hydrochloride, N^{ε}-lauroyllysine ethyl ester lactate, N^{ε}-lauroyllysine ethyl ester succinate, N^{ε} -lauroyllysine ethyl ester citrate, N^{ε}-lauroyllysine ethyl ester malate, N^{ε}-lauroyllysine ethyl ester phosphate, N^{ε}-lauroyllysine ethyl ester glutamate, N^{ε}-lauroyllysine ethyl ester aspartate, N^{ε} -lauroyllysine ethyl ester sulfate, N^{ε}-lauroyllysine methyl ester hydrochloride, N^{ε}-cocoyllysine ethyl ester hydrochloride, N^{ε} -myristoyllysine ethyl ester hydrochloride, N^{ε}-palmitoyllysine ethyl ester hydrochloride, N^{ε}-stearoyllysine ethyl ester hydrochloride, N^{ε}-oleoyllysine ethyl ester hydrochloride and the like; monoalkyl quaternary ammonium salts such as lauryl trimethyl ammonium chloride, myristyl trimethyl ammonium chloride, palmityl trimethyl ammonium chloride, stearyl trimethyl ammonium chloride, oleyl trimethyl ammonium chloride, eicosanyl trimethyl ammonium chloride, lauryl dimethyl benzyl ammonium chloride, stearyl dimethyl benzyl ammonium chloride, behenyl trimethyl ammonium chloride and the like; dialkyl quaternary ammonium salts such as dipalmityl dimethyl ammonium chloride, distearyl dimethyl ammonium chloride, di-hydrogenated tallow alkyl dimethyl ammonium bromide, di-hydrogenated tallow alkyl dimethyl ammonium salfate and the like; alkyl polyethenoxy ammonium salts such as distearyl polyethenoxy methyl ammonium chloride, dipalmityl polyethenoxy ethyl ammonium chloride, dipalmityl polyethenoxy hydroxy ethyl ammonium chloride, tristearyl polyethenoxy ammonium chloride, distearyl polyethenoxy ethyl ammonium ethyl sulfate, and the like.

Although the cosmetics or external preparations for skin in the present invention may be orally or non-orally administrated, the administration by applying them to the surface of the skin is preferable.

The compounding ratio of Compound (A) to Compound (B) or of Compound (C) to Compound (D) in the cosmetics or external preparations for skin in the present invention is usually 10/90-99/1, preferably 50/50-90/10 in weight ratio. In the case that the content of Compound (A) or Compound (C) is less than 10, the effect of mitigating the irritation and inflammation of the skin caused by Compound (B) or Compound (D) is insufficient. Contrary thereto, in the case that it exceeds 99, the chemical peeling effect, disinfectant effect, detergent effect, disinfectant and cleansing effect and other effect caused by Compound (B) or Compound (D) can not be exerted sufficiently.

The total content of Compound (A) and Compound (B) or that of Compound (C) and Compound (D) in the cosmetics or external preparations for skin in the present invention 0.01-90 % by weight, preferably 0.02-80 % by weight. When it is less than 0.01 % by weight, the chemical peeling effect, disinfectant effect, detergent effect, disinfectant and cleansing effect and other effect caused by Compound (B) or Compound (D) is not exerted sufficiently. When it exceeds 90 % by weight, it is not preferable because some sticky feeling occurs on the skin so that there is a problem in the practical use.

In using the above-mentioned components (A) and (B) or the above-mentioned components (C) and (D) which are incorporated into the cosmetics or external preparations for skin in the present invention, components which have been generally used as cosmetics or external preparations for skin may be, in addition to these components, added within the extent not to damage the effect of the present invention.

As the components which have been generally used as cosmetics or external preparations for skin, there mat be taken an antioxidant, an anti-inflammatory drug, an ultraviolet absorber, a whitening agent, a cell activating agent, a humectant, a metal chelating agent, an oily raw material, a surfactant, a solvent, a high molecular substance, a powdery substance, pigments, a perfume, a percutaneous absorption promoter, a steroid hormone, etc.

As examples of the anti-oxidant, there may be taken vitamin A substances, their derivatives and salts thereof such as retinol, dehydroretinol, retinol acetate, retinol palmitate, retinal, retinoic acid, vitamin A oil and the like; carotenoids and their derivatives thereof such as *α*-carotene, *β*-carotene, γ -carotene, cryptoxanthin, astaxanthin, fucoxanthin and the like; vitamin B substances, their derivatives and salts thereof such as pyridoxine, pyridoxal, pyridoxal-5-phosphoric acid ester, pyridoxamine and the like; vitamin C substances, their derivatives and salts thereof such as ascorbic acid, sodium ascorbate, ascorbyl stearate, ascorbyl palmitate, ascorbyl dipalmitate, magnesium ascorbate phosphate and the like; vitamin D substances, their derivatives and salts thereof such as ergocalciferol, chorecalciferol, 1, 25-dihydroxy-chorecalciferol and the like; vitamin E substances, their derivatives and salts thereof such as α-tocopherol, *β*-tocopherol, γ-tocopherol, δ -tocopherol, α-tocotrienol, *β*-tocotrienol, *γ*-tocotrienol, δ -tocotrienol, tocopherol acetate, tocophenol nicotinate and the like; Trolox, its derivative and salts thereof; dihydroxytoluene, butylhydroxytoluene, butylhydroxyanisole, dibutylhydroxytoluene, α-lipoic acid, dehydrolipoic acid; glutathion, its derivative and salts thereof; uric acid; erysorbic acid such as erysorbic acid, sodium erysorbate and the like; gallic acid, its derivative and salts thereof; such as gallic acid, propyl gallate and the like; rutin, its derivative and salts thereofsuch as rutin, α-glycosylrutin and the like; tryptophan, its derivative and salts thereof; histidine, its derivative and salts thereof; cysteine derivatives and salts thereof such as N-acetylcysteine, N-acetylhomocysteine, N-octanoylcysteine, N-acetylcysteine methyl ester and the like; cystine derivatives and salts thereof such as N, N'-diacetylcystine dimethyl ester, N, N'-dioctanoylcystine dimethyl ester, N, N'-dioctanoylhomocystine dimethyl ester and the like which are described in WO/0021925: carnosine, its derivative and salts thereof; homocarnosine, its derivative and salts thereof; anserine, its derivative and salts thereof; flavonoids such as carcinine, its derivative and salts thereof; di or tripeptides containing histidine and/or tryptophan and/or histamine, and salts thereof; flavonoids such as flavanone, flavone, anthocyanin, anthocyanidin, flavonol, quercetin, quercitrin, myricetin, fisetin, hamamelitannin; catechin, epicatechin, gallocatechin, epigallocatechin, epigallocatechin gallate and the like; tannic acid, caffeic acid, ferulic acid. protocatechuic acid, chalcone, oryzanol, carnisol, sesamol, sesamine, sesamolin, zingerrone, curcumine, tetrahydrocurcumine, clovamide, deoxyclovamide, Shogaol, capsaicin, vanillyl amide, ellagic acid, bromophenol, flavoglacin, melanoidine, riboflavin, riboflavin butyric acid ester, flavin mononucleotide, flavin adenine nucleotide, ubiquinone, ubiquinol, mannite, bilirubin, cholesterol, ebselen, selenomethionine, ceruloplasmin, transferring, lactferrin, albumin, bilirubin, superoxide dismutase, catalase, glutathione peroxidase, methallothionein, O-phosphono-pyridoxylidene rhodamine, N-(2-hydroxybenzyl)amino acid, its derivative and salts thereof described in US Patent No. 5,594,012, N-(4-pyridoxylmethylene)amino acid, etc.

As examples of the anti-inflammatory drug, there may be taken phenylbutazone, indomethacine, ibuprofen, ketoprofen, allantoin, guaiazulene, resorcin, hydrocortisone, prednisolone, methylprednisolone, dexamethasone, triamcinolone, triamcinolone acetonide, fludroxycortide, clobetasone, clobetasol and steroid esters thereof; ketal, acetal and hemiacetal derivative; flufenamic acid, bufexamac, naproxen, flurbiprofen, fenbufen, tenoxicam, piroxicam, mefenamic acid; salicylic acid, its derivative and salts thereof such as sodium salicylate, methyl salicylate, glycol salicylate and the like; D-panthenol, its derivative and salts thereof; glycyrrhizic acid, its derivative and salts thereof such as glycyrrhizic acid, methyl glycyrrhizate, dipotassium glycyrrhizate and the like; glycyrrhetic acid, its derivative and salts thereof such as glycyrrhetic acid, glyceryl glycyrrhetinate, stearyl glycyrrhetinate, glycyrrhetinyl stearate and the like; chondroitinsulfuric acid and salt thereof; ε-aminocaproic acid, dichlofenac sodium, tranexamic acid, diphenhydramine hydrochloride, chlorpheniramine maleate, ichthammol, γ-oryzanol, thianthol, sodium copper chlorophyllin, an extract of *Angelica keiskei,* arnica extract, aloe extract, *Bistorta* extract, *Curcuma* extract, *Hypericum* extract, *Chamomile* extract, glycyrrhiza (*Hemorocallis*) extract, Japanese Honeysuckle *(Lonicera japonica Thunb.)* extract, watercress (*Nasturium officinale*) extract, comfrey extract, *Acanthopanax sieboldianus* extract, an extract of *Salvia splendens,* bluish purple extract, *Perilla* extract, White brich extract, tea extract, *Angelica* extract, pot marigold extract, *Sambucus* extract, cattail (*Typha latifolia Linn.*) extract, *Sapindus* extract, wormwood (*Artemisia*) extract, *Eucalyptus* extract, *Astragalus* extract, zinc oxide, etc.

As examples of the ultraviolet absorber, there may be taken cinnamic acid ultraviolet absorbers such as 2-ethylhexyl p-methoxycinnamate, isopropyl p-methoxycinnamate, sodium p-methoxycinnamate, potassium p-methoxycinnamate, 2-ethoxyethyl p-methoxycinnamate, diethanolamine p-methoxyhydrocinnamate, glyceryl di(p-methoxycinnamic acid) mono-2-ethylhexanoate, octyl methoxycinnamate, methyl diisopropylcinnamate and the like; benzophenone ultraviolet absorbers such as 2-hydroxy-4-methoxybenzophenone, 2-hydroxy-4-methoxybenzophenone-5-salfuric acid, sodium salt of 2-hydroxy-4-methoxybenzophenone-5-sulfuric acid, dihydroxybenzophenone, 2, 4-dihydroxybenzophenone, 2, 2'-dihydroxy-4, 4'-dimethoxybenzophenone, 2, 2'-dihydroxy - 4-methoxybenzophenone, sodium salt of dihydroxydimethoxybenzophenone-5-sulfuric acid, 2, 2', 4, 4'-tetrahydroxybenzophenone, 2-hydroxy-4-n-octoxylbenzophenone and the like; benzoic acid ultraviolet absorbers such as p-aminobenzoic acid, sodium p-aminobenzoate, ethyl p-aminobenzoate, butyl p-aminobenzoate, 2-ethylhexyl p-aminobenzoate, amyl p-dimethylaminobenzoate, glyceryl p-aminobenzoate and the like; salicylic acid ultraviolet absorbers such as 2-ethylhexyl salicylate, triethanolamine salicylate, homomenthyl salicylate, dipropylen glycol salicylate, methyl salicylate, ethylene glycol salicylate, phenyl salicylate, amyl salicylate, benzyl salicylate, isopropylbenzyl salicylate, myristyl salicylate, potassium salicylate and the like; dibenzoylmethane ultraviolet absorbers such as 4-tert-butyl-4'-methoxydibenzoylmethane, 4-isopropyldibenzoylmethane, 4-methoxydibenzoylmethane, 4-tert-butyl-4'-hydroxydibenzoylmethane and the like; urocanic acid ultraviolet absorbers such as urocanic acid, ethyl urocanate and the like; menthyl-O-aminobenzoate, 2-phenyl-benzimidazole-5-sulfuric acid, 2-phenyl-5-methylbenzoxazole, 3-(4-methylbenzylidene)camphor, 2-ethtlhexyl-2-cyano-3, 3'-diphenylacrylate, 2-ethyl-2-cyano-3, 3'-diphenylacrylate, 2-(2'-hydroxy-5-methylphenyl)benzotriazole, methyl anthranilate, methyl anthranilate, ethyl anthranilate, menthyl anthranilate, 2, 4, 6-tris[4-(ethylhexyloxycarbonyl)anilino]-1,3,5-triazine, 3, 3' -(1, 4-phenylenedimethylidene)bis(7, 7-dimethyl-2-oxo-bicyclo[2, 2, 1] heptane-1-methanesulphonic acid) (Mexoryl SX), titanium oxide, zirconium oxide, cerium oxide,zinc oxide, etc.

As examples of the whitening agent, there may be taken tyrosinase inhibitor, endothelin antagonistic drug, α - MSH inhibitor, glabridine, glaburene, liquiritin, isoliquiritin, ellagic acid, its derivative and salts thereof; kojic acid, its derivative and salts thereof: hydroquinone such as arbutin, its derivative and salts thereof; cysteine, its derivative and salts thereof; vitamin C substances, their derivatives and salts thereof such as ascorbic acid, sodium sacorbate, ascorbyl stearate, ascorbyl palmitate, ascorbyl dipalmitate, magnesium ascorbate phosphate and the like; glutathione, its derivative and salts thereof; resorcin, its derivative and salts thereof; neoagarobiose, agarose oligosaccharide, asparagus extract, althaea extract, *Bistorta* extract, *Pisum sativum* extract, rosae fructus extract, scutellariae radix extract, *ononis spinose* extract, seaweeds extract, foutune firethorn (Pyracantha fortuneana) extract, glycyrrhiza (*Hemorocallis*) extract, *Rubis* extract, sophoras radix extract, muscovado extract, *Spatholobus suberectus Dunn* extract, *Acanthopanax sieboldianus* extract, an extract of wheat gerum, an extract of *Asiasarum sieboldii*, *Crataegus* (hawthorn) extract, *Cassia nomame Honda* extract, *Santalum* (peony) extract, white lily extract, *Gardenia jasminoides* extract, mori cortex extract, soybean extract, placenta extract, *Aralic* extract, tea extract, *Angerica* extract, molasses extract, wild rose extract, *Santalum* extract, grape seed extract, *Fagus* extract, flow de manita extract, hop extract, an extract of *Rosa rugosa var. plene Regal*, an extract of *Frunus japonica Thunb*, Saxifrage extract, an extract of *Coix lacryma-jobi, Siraitia grosvenori* extract, etc.

As examples of cell activating agent, there may be taken nucleic acid related substances, their derivatives and salts thereof such as deoxyribonucleic acid and salt thereof, adenylic acid derivative and salt thereof, ribonucleic acid and salt thereof, cyclic AMP, cyclic GMP, flavin adenine nucleotide, guanine, adenine, cytocine, thymine, xanthin, caffeine, theophylline and the like; vitamin A substances, their derivatives and salts thereof such as retinol, dehydroretinol, retinol acetate, retinol palmitate, retinal, retinoic acid, vitamin A oil and the like; carotenoids and derivatives thereof such as α-carotene, β -carotene, γ-carotene, cryptoxanthin, astaxanthin, fucoxanthin and the like; vitamin B substances, their derivatives and salts thereof such as pyridoxine, pyridoxal, pyridoxal-5-phosphoric acid ester, pyridoxamine and the like; vitamin C substances, their derivatives and salts thereof such as ascorbic acid, sodium sacorbate, ascorbyl stearate, ascorbyl palmitate, ascorbyl dipalmitate, magnesium ascorbate phosphate and the like; vitamin D substances, their derivatives and salts thereof such as ergocalciferol, chorecalciferol, 1, 25-dihydroxy-chorecalciferol and the like; vitamin E substances, their derivatives and salts thereof such as α-tocopherol, *β*-tocopherol, γ-tocopherol, δ -tocopherol, α-tocotrieriol, β-tocotrienol, *γ*-tocotrienol, δ -tocotrienol, tocopherol acetate, tocopherol nicotinate and the like; Trolox, its derivative and salts thereof; Japanese cypress thymol, cepharanthine, α - linolenic acid, γ - linolenic acid, eicosapentaenoic acid, its derivative and salts thereof; organic acids selected from glycolic acid, succinic acid, lactic acid and salicylic acid, their derivatives and salts thereof; estradiol, its derivative and salts thereof; silk protein, its hydrolized product and derivatives thereof; hemoglobin or its hydrolyzed product; lactoferrin or its hydrolyzed product; royal jelly, extracts derived from animals such as placenta extract, an extract of young cattle blood, an extract of deproteinized serum, spleen extract, egg components, crest extract, shell extract, shell meat extract, mollusk extract, fish extract and the like; extracts derived from microorganism such as fermentation metabolite and the like; plant extracts such as asparagus extract, apricot extract, ginkgo extract, phellodendron bark extract, barley extract, an extract of *Panax schin-seng*, orange extract, kiwi fruit extract, cucumber extract, *Lentinus* extract, *Equisetum* extract, *Swertia* extract, zizyphi fructus extract, *Capsicum* extract, cayenne pepper extract, carrot (*Daucus*) extract, garlic extract, *Pachy ma hoelen* extract, grape seed extract, *Fagus* sprout extract, peach extract, *Eucalyptus* extract, *Fomes japonioucus* extract, lettuce extract, lemon extract, rosemary extract, or the like.

As examples of the humectant, there may be taken mucopolysaccharides or salts thereof; protein or protein hydrolyzed product, their derivatives and salts thereof; phospholipid derived from soybean or egg; glycolipid, ceramide, mucin, honey, saccharides and derivatives thereof such as erythritol, maltose, maltitol, xylitol, xylose, pentaerythritol, fructose, dextrin, and the like; acidic polysaccharides such as hyaluronic acid and the like; urea, amino acids, their derivatives and salts thereof such as asparagine, aspartic acid, alanine, arginine, isoleucine, ornithine, glutamine, glycine, glutamic acid, cysteine, cystine, citrulline, threonine, serine, tyrosine, triptophan, theanine, valine, histidine, hydroxylysine, hydroxyproline, pyrrolidonecarboxylic acid, proline, phenylalanine, methionine, lysine, and the like; D-panthenol, whey protein, *Angellica* keiskei extract, avocado (*Persea*) extract, almond extract, althaea extract, arunica extract, aloe extract, strawberry extract, locust extract, *Streptochaeta, Oryza* extract, *artemisia capillaris flos.* extract, *Foeniculum* extract, *Curcuma* extract, an extract of *Malva sylvestris,* perilla oil, scutellariae radix extract, Coptis extract, *Lamium* extract, *Hypericum* extract, ononis spinose extract, olive oil, seaweeds extract, cacao butter, camonile extract, *Avena* extract, *Garcinia cambogia* extract, glycyrrhiza *(Hemorocallis)* extract, *Rubus palmatus* extract, *Hedera* extract, Japanese Honeysuckle (*Lonicera japonica Thunb.*) extract, *Gardenia* extract, *Sasa* extract, grapefruit extract, sophoras radix extract, watercress *(Nasturium officinale)* extract, *Gentiana* extract, *Geranium* extract, *Arctium* extract, *Clematis apiifolia* extract, *Sesamum* extract, wheat extract, comfrey extract, an extract of *Asiasarum sieboldii,* cactus extract, soapwort extract, *Salvia splendens* extract, *Crataegus* extract, shea butter, *Perilla* extract, rehmanniae radix extract, *Spiraea* extract, peony extract, *Zingiber* extract, white birch extract, *Malva* extract, *Cnidium officinale* extract, mori cortex extract, soybean extract, *Thymus vulgaris* extract, tea extract, *Camellia* extract, *Angelica* extract, corn (*Tripsacum, Zea*) extract, *Cordyceps* extract, *Houttuynia* extract, torumenchira extract, *Lupinus perennis* extract, ophiopogonus tuber extract, parsley extract, *Mentha* extract, *Mentha viridis Linn.* extract, *Mentha piperita* extract, witch hazel (*Hamamelis Virginiana)* exract, *Rosa* extract, *Chamaecyparis* extract, *Herinthus* extract, grape *(Vitis)* extract, Bucher's room extract, prune extract, *Luffa* extract, Tilia extract, Paeonia extract, hop extract, jojoba oil, borage oil, macademia nuts extract, *Pinus* extract, quince extract, horse chestnut extract, *Sapindus* extract, *Lithospermum* extract, Meadowfoam Seed Oil, Melissa (*Melissa officinalis*) extract, *Rodgersia* extract, *Saxifrage* extract, citron extract, *Lilium* extract, an extract of *Coix lacryma-jobi,* lime extract, arhat extract, lavender extract, apple (*Malus*) extract, *Gentiana* extract, an extract of Chinese milk vetch, *Sanguisorba* extract, alkali simple spring water, deep water, etc.

As examples of the metal chelating agent, there may be taken malic acid, citric acid, salicylic acid, tartaric acid, gluconic acid, phytic acid, their derivatives and salts thereof; ethylenediamine-tetraacetic acid, its derivative and salts thereof; diethlenetriamine-pentaacetic acid, its derivative and salts thereof; N-caboxymethylaspartic acid, its derivative and salts thereof; N-caboxymethylglutamic acid, its derivative and salts thereof; N, N-bis (caboxymethyl)aspartic acid, its derivative and salts thereof; N, N-bis(caboxymethyl)glutamic acid, its derivative and salts thereof; N, N-bis(succinic acid)ethylenediamine, its derivative and salts thereof; desferrioxamine, o-phenanthroline, transferrin, ferritin, lactoferrin, caffeic acid, maltitol, purpurogallin, pyrogallol, sodium polyphosphate, sodium methaphosphate, sodium hexamethaphosphate, etc.

As examples of the oily raw materials, there may be taken oils and fats such as animal and vegetable oils and the like; waxes such as lanolin and the like; hydrocarbons such as paraffin, and the like; higher alcohols such as cetanol and the like; higher fatty acids such as stearic acid and the like; sterols; phosphotides such as lechithin and the like; synthetic esters such as myristic acid and the like; metal soaps, silicone oil, perfluoropolymer, perfluoropolyether, etc.

As examples of the surfactant, there may be taken ampholytic surfactants, nonionic surfactants, emulsifying agents, solubilizing agents, and the like.

As examples of the solvent, there may be taken lower alcohols such as ethanol and the like, ethers, glycerins, liquid nonionic surfactants, liquid oily raw materials, other organic solvents, water, etc.

As examples of the higher molecular substances, there may be taken polyamino acids and derivatives thereof such as polyaspartic acid, ε -polylysine, γ -polyglutamic acid and the like; natural high molecular compounds such as collagen, elastin and the like; semi-synthetic high molecular compounds such as partially deacetylated chitin and the like, synthetic high molecular compounds such as carboxymethylcellulose, etc.

As examples of the powdery substances, there may be taken inorganic pigments such as talc and the like, functional pigments such as synthetic mica and the like, hybrid fine powders, pearl-lustrous pigments such as titanium oxide-coated mica and the like, photochromic pigments, high molecular powders such as nylon polymer and the like, organic pigments such as N-ε-lauroyllysine, etc.

As examples of the colorants, there may be taken legal 1st-class tar pigments, legal 2nd-class tar pigments, legal 3rd-class tar pigments, hair dyes, natural colorants, mineral colorants, etc.

As examples of the perfumes, there may be taken animal perfumes such as musk and the like, plant perfumes such as jasmine oil and the like, synthetic perfumes such as α -amylcinnamaldehyde and the like, mixed perfumes, etc.

As examples of the percutaneous absorption promotors, there may be taken 2-pyrrolidone, 1-hexanol, 1-octanol, 1-decanol, 1-menthol, sodium lauryl sulfate, isopropyl myristate, n-hexyl acetate, oleic acid, etc.

As the steroid hormone, there may be taken 21-acetoxypregnenolone, alclometasone, algestone, amcinonide, beclomethasone, betamethasone, budesonide, chloroprednisone, clobetasone, clocortolone, cloprednol, corticosterone, cortisone, cortisol, deflazacort, desonide, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, formocortal, halcinonide, halometasone, halopredone acetate, hydrocortamate, hydrocortisone, hydrocortisone phosphate, hydrocortisone-21-succinate sodium salt, hydrocortisone tebutate, mazipredone, medrysone, meprednisone, methylprednisolone, mometasone furoate, paramethasone, prednicarbate, prednisolone-21-diethylaminoacetate, prednisolone sodium phosphate, prednisolone sodium succinate, prednisolone sodium -21-m-sulfobenzoate, prednisolone-21-stearoylglycolate, prednisolone tebutate, prednisolone-21-trimethylacetate, predonisone, prednival, prednylidene, prednylidene-21-diethylaminoacetate, tixocortol, triamcinolone, triamcinolone acetonide, triamcinolone benetonide, triamcinolone hexaacetonide, fluticasone, etc.

The dosage form of the cosmetics or external preparations for skin containing the above-described Components (A) and (B) or the above-described Components (C) and (D) in the present invention is not limited particularly, and any dosage form such as solution, paste, gel, solid, powder or the like can be adopted. Also, the cosmetics or external preparations for skin involving in the present invention can be used as or for oil, lotion, cream, milky lotion, gel, shampoo, hair rinse, hair conditioner, enamel, foundation, lipstick, face powder, pack, ointment, tablet, injection, granule, capsule, perfumed water, powder, eau de Cologne, dental paste, soap, aerosol, creasing form and the like, and further as or for skin aging preventive or alleviative, skin inflammation preventive or alleviative, bath agent, hair tonic, skin vitalizing lotion, sunburn preventive, preventive or alleviative of photosensitivity such as xerodrma pigmentosum or solar urticaria, preventive or alleviative of photoallergy, preventive or alleviative of optical xeroderma pigmentosum, or preventive or alleviative of rough skin caused by injury, chaps, cleft or the like, disinfectant, bactericide, insecticide, disinfestant, keratolytic, epiderm peeling agent, preventive or alleviative of acne vulgaris, preventive or alleviative of various skin diseases such as keratosis, xeroderma, ichthyosis and alphos, etc.

Furthermore, other ordinarily employed component in cosmetics or external preparations for skin can be added to the cosmetics or external preparations for skin containing the above-described Components (A) and (B) or the above-described Components (C) and (D) within an extent not impairing the effect of the present invention. As such other ordinarily employed component in cosmetics or external preparations for skin, there may be taken antiseptic, discoloration preventives, buffers, medicaments for acne vulgaris, antidandruffs or antipruritics, antiperspirants or antibromics, antipyrotics, acaricides or pediculicides, keratin softeners, medicaments for xeroderma, virucides, hormones, vitamins, amino acids, peptides, proteins, astringents, coolants or stimulators, components derived from animal and plants, antibiotics, hair growth promoters, etc.

### The Best Mode For Carrying Out The Invention

The present invention is illustrated in more detail with reference to the following Examples (Synthesis Examples, Test Examples and Formulation Examples) but it does not restricted to these Examples. Incidentally, the amount incorporated of each component is indicated by weight %.

### Synthesis Example 1: Synthesis of cystine dialkyl ester derivatives

To 25 ml of acetonitrile were added successively 0.50 g of L-cystine dimethyl ester dihydrochloride, 0.55 g of n-pentanoic anhydride and 0.31 g of triethylamine, followed by stirring overnight at room temperature. The reaction solution was concentrated, and the crude crystals thus obtained were purified by high-performance liquid chromatography (HPLC separation with an apparatus for high-performance liquid chromatography manufactured by Hitachi, Ltd. wherein "Inertsil ODS-3 Column" (a product of GL Science Co., Ltd.) is used), whereby 0.38 g of N, N'-di (n-valeryl)-L-cystine dimethyl ester was obtained. Similarly, various N, N'-diacyl-L-cystine dialkyl esters for use in Formulation Examples were obtained.

### Synthesis Example 2: Synthesis of cystine diamide derivatives

To 7 ml of acetonitrile were added successively L-cystine diamide dihydrochloride, 0.21 g of n-hexanoic anhydride and 0.10 g of triethylamine, followed by stirring overnight at room temperature. The reaction solution was concentrated, and the crude crystals thus obtained were purified by the similar high-performance liquid chromatography as in Synthesis Example 1, whereby 0.18 g of N, N'-di (n-hexanoyl)-L-cystine diamide was obtained. Similarly, various N, N'-diacyl -L-cystine diamides for use in Formulation Examples were obtained.

### Test Example 1: Test on the primary irritation of the skin

Each back portion of rabbits (New Zealand White species, male) was shaved and each of the test compounds was applied thereto. Each of the test compound-applied portions was blocked for 24 hours. After 24, 48, 72 and 168 hours from the time of applying the test compound, the judgments were conducted for the erythema and edema appeared on the test compound-applied portion based on their standards indicated in table 1. The average evaluation value in each time at which the judgment was conducted was calculated (the value obtained by dividing the combined value of the evaluation value for erythema and that for edema by the number of animal used in the test). In addition, the average evaluation value in each time at which the judgment was conducted was all added up and the total value was divided by the number of judgment (4 times) to make the evaluation value of the primary irritation. The results are shown in table 2.

**Table 1**

| Evaluation Value | Evaluation standards For erythema | Evaluation standards For edema |
|---|---|---|
| 0 | No erythema | No edema |
| 1 | Very slight erythema | Very slight edema |
| 2 | Apparent erythema | Apparent edema |
| 3 | From moderate erythema to strong one | Moderate edema |
| 4 | Strong erythema | Strong edema |

**Table 2**

| Test compound (concentration) | Evaluation Value For irritation |
|---|---|
| Sodium lauryl sulfate (5%) | 6.4 |
| Sodium lauryl sulfate (5%) + N, N'-diacetyl-L-cystine dimethyl ester (30%) | 5.2 |
| Lactic acid (70%) | 1.4 |
| Lactic acid (70%) + N, N'-diacetyl-L-cystine dimethyl ester (30%) | 0.5 |
| Benzalkonium chloride (3%) | 5.3 |
| Benzalkonium chloride (3%) + N, N'-diacetyl-L-cystine dimethyl ester (30%) | 4.8 |
| Sodium lauryl sulfate (5%) | 6.6 |
| Sodium lauryl sulfate (5%) + N, N'-dioctanoyl-L-cystine dimethyl ester (5%) | 6.0 |
| Benzalkonium chloride (3%) | 5.3 |
| Benzalkonium chloride (3%) + N, N'-dioctanoyl-L-cystine dimethyl ester (5%) | 3.2 |
| Stearyl trimethyl ammonium chloride (3%) | 3.7 |
| Stearyl trimethyl ammonium chloride (3%) + N, N'-dioctanoyl-L-cystine dimethyl ester (5%) | 2.0 |

It can be understood that N, N'-diacetyl-L-cystine dimethyl ester or N, N'-dioctanoyl-L-cystine dimethyl ester, being correspondingly Compound (A) or Compound (C) involving in the present invention when used in combination with lactic acid, glycolic acid, benzalkonium chloride, sodium lauryl sulfate or stearyl trimethyl ammonium chloride, being Compound (B) or Compound (D) involving in the present invention mitigates the primary irritation of the skin in comparison with the case that lactic acid, glycolic acid, benzalkonium chloride, sodium lauryl sulfate or stearyl trimethyl ammonium chloride was used each singly, as shown in table 2.

Next, Formulation Examples 1-16 of various preparations are shown. These preparations were prepared according to the conventional method. Incidentally, the amount incorporated of each component is indicated by weight %.

### Example 2: Test in human on function of inhibiting the irritation induced by an organic acid

A series of tests were conducted by seven adult men with respect to the function of inhibiting the irritation of the skin induced by an organic acid. There were prepared Solution A of only glycolic acid dissolved in a 25 % aqueous ethanol solution (the concentration of glycolic acid: 10 %) and Solution B of glycolic acid and N, N'-diacetyl-L-cystine dimethyl ester dissolved in a 25 % aqueous ethanol solution (the concentration of glycolic acid: 10 %, the concentration of N, N'-diacetyl-L-cystine dimethyl ester: 10 %). At that time, the pH of these two solutions was adjusted so as to be identical each other. After all of the panelists washed their faces, one of the solutions was applied to each right cheek of the their faces with a cotton bud. The irritation which each of the panelists felt at the solution-applied portion was recorded as the score every a minute after a minute from the time of applying the solution for 10 minutes based on its evaluation standards shown below. And then, the other solution was applied to each left cheek of the their faces with a cotton bud and the irritation which each of the panelists felt at the solution-applied portion was similarly recorded as the score. In addition, the function of the test compound (N, N'-diacetyl-L-cystine dimethyl ester) for inhibiting the irritation was judged based on the judging standards shown below.

There were prepared Solution C of only glycolic acid dissolved in a 25 % aqueous ethanol solution (the concentration of glycolic acid: 20 %) and Solution D of glycolic acid and N, N'-dioctanoyl-L-cystine dimethyl ester dissolved in a 25 % aqueous ethanol solution (the concentration of glycolic acid: 20 %, the concentration of N, N'-dioctanoyl-L-cystine dimethyl ester: 5 %), and the function of N, N'-dioctanoyl-L-cystine dimethyl ester for inhibiting the irritation was judged by similar manner as in the above.

The test results of N, N'-diacetyl-L-cystine dimethyl ester are shown in table 3 while that of N, N'-dioctanoyl-L-cystine dimethyl ester are shown in table 4.

### Evaluation standards

- Score: 0: to feel no irritation
- Score: 1: to feel very slight irritation (discontinuously sensible)
- Score: 2: to feel slight irritation (continuously sensible)
- Score: 3: to feel moderate irritation (with uncomfortable feeling)
- Score: 4: to feel heavy irritation (very uncomfortable and unendurable)

### Judging standards

- Judgment:: ○ the cumulative score value of Solution B ( or Solution D) is lower by at least 5 than that of Solution A ( or Solution C)
- Judgment:: Δ lower by at least 1 but at most 4 in the same comparison
- Judgment:: X lower by less than 1 in the same comparison

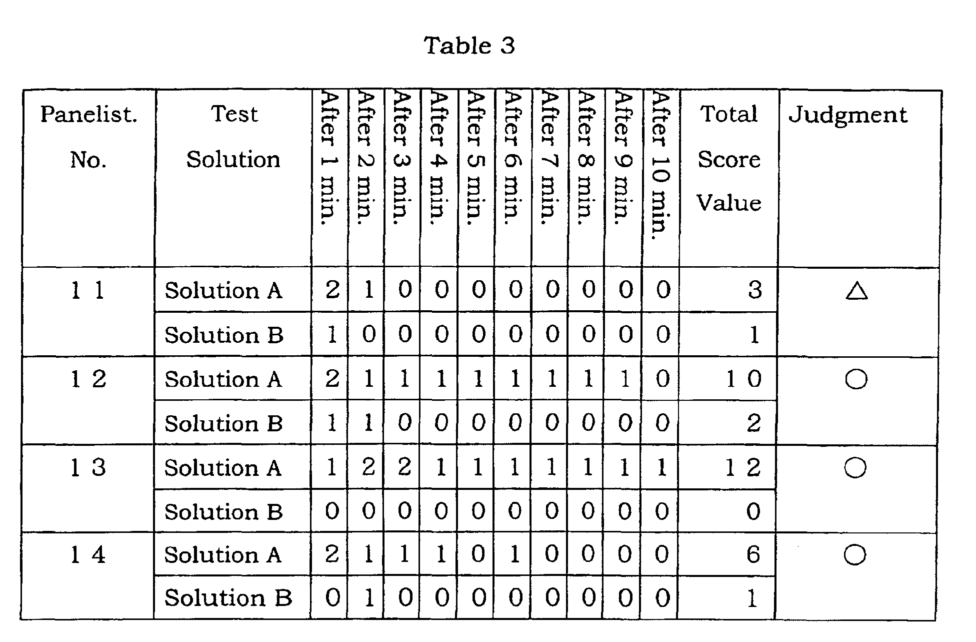

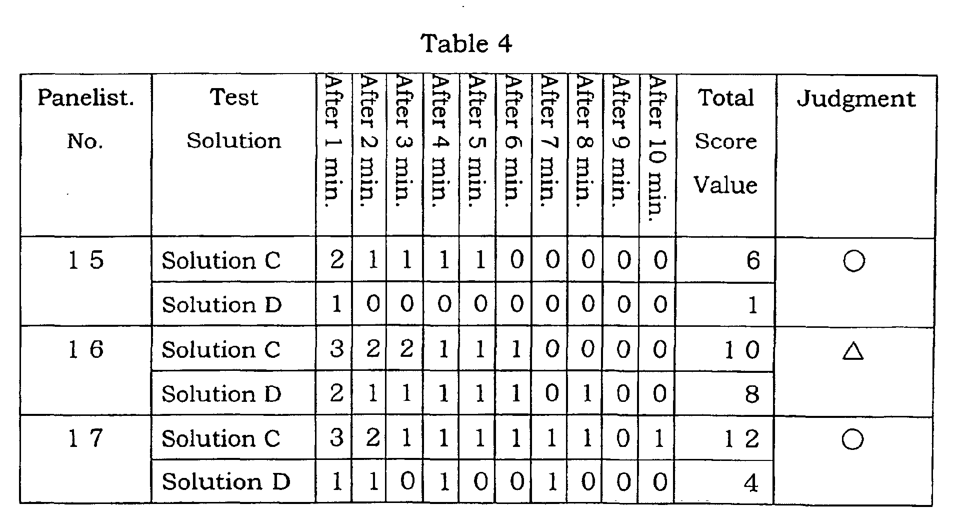

### Example 3: Test in human on function of inhibiting the irritation induced by an organic acid

A series of tests were conducted by four adult men with respect to the function of inhibiting the irritation of the skin induced an organic acid. There were prepared a solution of glycolic acid dissolved in a 25 % aqueous ethanol solution (the concentration of glycolic acid: 10 %) and a test solution of N, N'-diacetyl-L-cystine dimethyl ester dissolved in the same solvents (the concentration of N, N'-diacetyl-L-cystine dimethyl ester: 10 %). A solution of only the solvent was separately prepared as the control solution. After all of the panelists washed their faces, either test solution or control solution was applied to each right cheek of the their faces with a cotton bud. After additional 3 minutes, the glycolic acid solution was applied to the same portion as in the above and the irritation which each of the panelists felt was recorded as the score every a minute after a minute from the time of applying the glycolic acid solution for 10 minutes based on its evaluation standards shown below. And then, either test solution or control solution which was not applied initially was applied to each left cheek of the their faces with a cotton bud. After additional 3 minutes, the glycolic acid solution was applied to the same portion and the irritation which each of the panelists felt at the applied portion was similarly recorded as the score every a minute after a minute from the time of applying the glycolic acid solution for 10 minutes. In addition, the function of the test compound (N, N'-diacetyl-L-cystine dimethyl ester) for inhibiting the irritation was judged based on the judging standards shown below.

The test results of N, N'-diacetyl-L-cystine dimethyl ester are shown in table 5.

### Evaluation standards

- Score: 0: to feel no irritation
- Score: 1: to feel very slight irritation (discontinuously sensible)
- Score: 2: to feel slight irritation (continuously sensible)
- Score: 3: to feel moderate irritation (with uncomfortable feeling)
- Score: 4: to feel heavy irritation (very uncomfortable and unendurable)

### Judging standards

- Judgment:: ○ the cumulative score value of test solution is lower by at least 5 than that of control solution
- Judgment:: Δ lower by at least 1 but at most 4 in the same comparison
- Judgment:: × lower by less than 1 in the same comparison

**Table 5**

| Panelist No. | | After 1 min. | After 2 min. | After 3 min. | After 4 min. | After 5 min. | After 6 min. | After 7 min. | After 8 min. | After 9 min. | After 10 min. | Total score value | Judgment |
|---|---|---|---|---|---|---|---|---|---|---|---|---|---|
| 1 | Control solution | 3 | 2 | 2 | 2 | 1 | 1 | 0 | 0 | 0 | 0 | 11 | ○ |
| | Test solution | 2 | 1 | 0 | 0 | 0 | 0 | 0 | 0 | 0 | 0 | 3 | |
| 2 | Control solution | 2 | 1 | 1 | 1 | 1 | 0 | 0 | 0 | 0 | 0 | 6 | ○ |
| | Test solution | 1 | 0 | 0 | 0 | 0 | 0 | 0 | 0 | 0 | 0 | 1 | |
| 3 | Control solution | 2 | 2 | 2 | 2 | 1 | 1 | 1 | 1 | 1 | 0 | 13 | ○ |
| | Test solution | 1 | 1 | 1 | 1 | 1 | 1 | 1 | 0 | 0 | 0 | 7 | |
| 4 | Control solution | 2 | 3 | 3 | 2 | 1 | 1 | 1 | 0 | 0 | 0 | 13 | ○ |
| | Test solution | 0 | 1 | 1 | 1 | 1 | 0 | 0 | 0 | 0 | 0 | 4 | |

As shown in table 5, N, N'-diacetyl-L-cystine dimethyl ester exhibited inhibitory function in human toward irritation caused by glycolic acid (the concentration: 10 %).

### Example 4: Test in human on function of inhibiting the irritation induced by an organic acid

A series of tests were conducted by three adult men with respect to the function of inhibiting the irritation of the skin induced an organic acid. There were prepared a 30 % aqueous glycolic acid solution A (the concentration of glycolic acid: 20 %), a solution B of N, N'-diacetyl-L-cystine dimethyl ester dissolved in a 30 % aqueous glycolic acid solution (the concentration of N, N'-diacetyl-L-cystine dimethyl ester: 1 %), and a 30 % aqueous glycerin solution C. After all of the panelists washed their faces, solution B (or solution C) was applied to each right cheek (or each left cheek) of the their faces with a cotton bud. After 4 minutes from the time of applying the solution, the superfluous liquid adhered to the skin was wiped up, and the solution A was applied to solution B (or solution C)-applied portion. The irritation which each of the panelists felt at the solution-applied portion was recorded as the score every a minute after a minute from the time of applying the solution for 5 minutes based on its evaluation standards shown below. In addition, the function of the test compound (N, N'-diacetyl-L-cystine dimethyl ester) for inhibiting the irritation was judged based on the judging standards shown below.

The test results of N, N'-diacetyl-L-cystine dimethyl ester are shown in table 6.

### Evaluation standards

- Score: 0: to feel no irritation
- Score: 1: to feel very slight irritation (discontinuously sensible)
- Score: 2: to feel slight irritation (continuously sensible)
- Score: 3: to feel moderate irritation (with uncomfortable feeling)
- Score: 4: to feel heavy irritation (very uncomfortable and unendurable)

### Judging standards

- Judgment:: ○ the cumulative score value of Solution B is lower by at least 5 than that of Solution C
- Judgment:: Δ lower by at least 1 but at most 4 in the same comparison
- Judgment:: × lower by less than 1 in the same comparison

**Table 6**

| Panelist No. | Test Solution | After 1 min. | After 2 min. | After 3 min. | After 4 min. | After 5 min. | Total Score Value | Judgment |
|---|---|---|---|---|---|---|---|---|
| 1 | Solution B | 1 | 1 | 1 | 0 | 0 | 3 | Δ |
| | Solution C | 1 | 1 | 1 | 1 | 1 | 5 | |
| 2 | Solution B | 0 | 2 | 1 | 0 | 0 | 3 | ○ |
| | Solution C | 3 | 3 | 3 | 3 | 3 | 15 | |
| 3 | Solutions | 0 | 0 | 0 | 0 | 0 | 0 | ○ |
| | Solution C | 2 | 1 | 1 | 1 | 1 | 6 | |

As shown in table 6, N, N'-diacetyl-L-cystine dimethyl ester exhibited inhibitory function in human toward irritation caused by glycolic acid (the concentration: 20 %).

### Example 5: Test in human on function of inhibiting erythema and irritation induced by an organic acid

A series of tests were conducted by an adult man with respect to the function of inhibiting the skin erythema and irritation induced by an organic acid. A test solution of N, N'-diacetyl-L-cystine dimethyl ester dissolved in a 25 % aqueous ethanol solution (the concentration of N, N'-diacetyl-L-cystine dimethyl ester: 10 %) was prepared. A solution of only the solvents was separately prepared as control solution. 30 Times of tape stripping were conducted on the inward portion of the panelist's forearm. To the portion which the tape stripping procedures have been conducted was applied ② test solution (or control solution) in addition to ① control (any solvent is not applied). After 4 minutes, to each of the portions (① control portion (both forearms) ② test solution-applied portion and ③ control solution-applied portion) was applied protopic ointment (tacrolimus: 1mg/g), and the erythema and irritation appeared on each of the portions after an additional 5 minutes were evaluated. Slight erythema was confirmed on the control portion and control solution-applied portion while any erythema was not recognized on test solution-applied portion. In addition, irritation was sensible in control solution-applied portion while any irritation was not sensible in test solution-applied portion and the control portion. In the skin where the barrier function of the skin was reduced by tape stripping (30 times), erythema and irritation caused by the protopic ointment were suppressed by N, N'-diacetyl-L-cystine dimethyl ester.

### Formulation Example 1: Ointment

| | |
|---|---|
| N, N'-di (n-decanoyl)-L-cystine dimethyl ester | 1.0 % |
| Benzalkonium chloride | 0.1 % |
| Urea | 20.0 % |
| White Vaseline | 15.0 % |
| Light liquid paraffin | 6.0 % |
| Cetanol | 3.0 % |
| Stearyl alcohol | 3.0 % |
| Glyceryl monostearate | 5.0 % |
| Perfume | q.s. |
| Antiseptic | q.s. |
| Buffer | 1.0 % |
| Purified water | Balance |

### Formulation Example 2: Ointment

| | |
|---|---|
| N, N'-di (n-lauroyl)-L-cystine dimethyl ester | 2.0 % |
| White Vaseline | 25.0 % |
| Stearyl alcohol | 20.0 % |
| Propylene glycol | 12.0 % |
| Polyoxyethylene hardened castor oil | 4.0 % |
| Glycerin monostearate | 1.0 % |
| Glycolic acid | 1.0 % |
| Trichloroacetic acid | 1.0 % |
| Antiseptic | q. s. |
| Perfume | q.s. |
| Purified water | Balance |

### Formulation Example 3: Lotion

| | |
|---|---|
| N, N'-di (n-valeryl)-L-cystine dimethyl ester | 3.0 % |
| Glycolic acid | 5.0 % |
| Glycerin | 3.0 % |
| Sorbitol | 2.0 % |
| Polyoxyethylene (20) oleyl ether | 1.0 % |
| Ethanol | 15.0% |
| Zinc p-phenol sulphonate | 0.2% |
| Buffer | 0.1 % |
| Perfume | 0.2 % |
| Antiseptic | q.s. |
| Purified water | Balance |

### Formulation Example 4: Lotion

| | |
|---|---|
| N, N'-di (n-propionyl)-L-cystine dimethyl ester | 0.5 % |
| Citric acid | 1.0 % |
| Urea | 4.0% |
| Salicylic acid | 2.0 % |
| Lactic acid | 2.0 % |
| Glycerin | 2.0 % |
| Betaine | 2.0 % |
| Hyaluroinic acid | 0.1 % |
| Ethanol | 15.0 % |
| Buffer | 0.1% |
| Perfume | 0.2 % |
| Antiseptic | q.s. |
| Purified water | Balance |

### Formulation Example 5: Lotion

| | |
|---|---|
| N, N'-di (n-hexanoyl)-L-cystine dimethyl ester | 0.5 % |
| Lactic acid | 0.1 % |
| Fruit acid | 0.1 % |
| Glycerin | 4.0 % |
| Kaolin | 1.0 % |
| Caramine | 0.7 % |
| Camphor | 0.2 % |
| Ethanol | 14.0 % |
| Perfume | q.s. |
| Purified water | Balance |

### Formulation Example 6: Cream

| | |
|---|---|
| N, N'-di (n-butyryl)-L-cystine dimethyl ester | 1.0 % |
| Resorcinol | 0.1 % |
| Kojic acid | 1.0 % |
| Stearic acid | 2.0 % |
| Polyoxyethylene (25) cetyl ether | 3.0 % |
| Glyceryl monostearate | 2.0 % |
| Octyl dodecanol | 10.0 % |
| Cetanol | 6.0 % |
| Reduced lanolin | 4.0 % |
| Squalane | 9.0 % |
| 1, 3-Butylene glycol | 6.0 % |
| Polyethylene glycol (1500) | 4.0 % |
| Antiseptic | q.s. |
| Perfume | q.s. |
| Purified water | Balance |

### Formulation Example 7: Cream

| | |
|---|---|
| N, N'-di (n-octanoyl)-L-cystine dimethyl ester | 1.0 % |
| Glycolic acid | 2.0 % |
| Solid paraffin | 5.0 % |
| Bees wax | 10.0 % |
| Vasseline | 15.0 % |
| Liquid paraffin | 41.0 % |
| 1, 3-Butylene glycol | 4. 0 % |
| Glyceryl monostearate | 2.0 % |
| Polyoxyethylene (20) sorbitan monolaurate | 2.0 % |
| Borax | 0.2 % |
| Antiseptic | q.s. |
| Perfume | q.s. |
| Antioxidant | q. s. |
| Purified water | Balance |

### Formulation Example 8: Cream

| | |
|---|---|
| N, N'-di (n-valeryl)-L-cystine dimethyl ester | 5.0 % |
| Propylene glycol | 6.0 % |
| Dibutyl phthalate | 19.0 % |
| Stearic acid | 5.0 % |
| Glyceryl monostearate | 5.0 % |
| Sorbitan monostearate | 12.0 % |
| Polyethylene sorbitan monostearate | 38.0 % |
| Glycolic acid | 1.0 % |
| Trichloroacetic acid | 1.0 % |
| Chelating agent | q.s. |
| Antiseptic | q.s. |
| Perfume | q. s. |
| Purified water | Balance |

### Formulation Example 9: Milky lotion

| | |
|---|---|
| N, N'-diacetyl-L-cystine | 2.0 % |
| N^{α}-cocoylarginine ethyl ester DL-pyrrolidone carboxylate | 0.1 % |
| Retinol | 0.1% |
| Bees wax | 0.5 % |
| Vasseline | 2.0 % |
| Glyceryl monostearate | 1.0 % |
| Polyethylene glycol monooleate | 1.0 % |
| Methyl polysiloxane | 2.0 % |
| Cetanol | 1.0 % |
| Squalane | 6.0% |
| Carboxyvinyl polymer | 0.5 % |
| 1, 3-Butylene glycol | 4.0 % |
| Ethanol | 5.0 % |
| Antiseptic | q.s. |
| Perfume | q.s. |
| Antioxidant | q.s. |
| Purified water | Balance |

### Formulation Example 10: Milky lotion

| | |
|---|---|
| N, N'-di (n-hexanoyl)-L-cystine dimethyl ester | 1.0 % |
| Lactic acid | 2.0 % |
| Stearyl alcohol | 0.5 % |
| Hardened palm oil | 3.0 % |
| Liquid paraffin | 35.0 % |
| Dipropylene glycol | 6.0 % |
| Polyethylene glycol (400) | 4.0 % |
| Sorbitan sesquioleate | 1.6 % |
| Polyoxyethylene (20) oleyl ether | 2.4 % |
| Carboxyvinyl polymer | 1.5 % |
| Potassium hydroxide | 0.1 % |
| Chelating agent | q.s. |
| Antiseptic | q.s. |
| Perfume | q.s. |
| Purified water | Balance |

### Formulation Example 11: Gel

| | |
|---|---|
| N, N'-diacetyl-L-cystine diethyl ester | 0.5 % |
| Liquid paraffin | 12.0 % |
| Glyceryl tri(2-ethylhexanate) | 50.0 % |
| Sorbit | 10.0 % |
| Polyethylene glycol (400) | 5.0 % |
| Lactic acid | 1.0 % |
| Acylmethyl taurine | 4.0 % |
| Polyoxyethylene (20) isocetyl ether | 10.0 % |
| Perfume | q.s. |
| Antiseptic | q.s. |
| Purified water | Balance |

### Formulation Example 12: Vitalizing lotion

| | |
|---|---|
| N, N'-di(n-heptanoyl)-L-cystine dimethyl ester | 0.5 % |
| Fruit acid | 0.5 % |
| Dipropylene glycol | 5.0 % |
| Polyethylene glycol (400) | 5.0 % |
| Ethanol | 10.0 % |
| Carboxyvinyl polymer | 0.5 % |
| Sodium alginate | 0.5 % |
| Potassium hydroxide | 0.2 % |
| Polyoxyethylene (20) sorbitan monostearate | 1.0 % |
| Sorbitan monooleate | 0.5 % |
| Oleyl alcohol | 0.5 % |
| Placenta extract | 0.2 % |
| dl-tocopherol acetate | 0.2 % |
| Perfume | q.s. |
| Antiseptic | q.s. |
| Discoloration inhibitor | q.s. |
| Purified water | Balance |

### Formulation Example 13: Pack

| | |
|---|---|
| N, N'-diacetyl-L-cystine diisopropyl ester | 3.0 % |
| Isopropanol | 2.0 % |
| Polyvinyl alcohol | 15.0 % |
| Carboxymethylcellulose | 5.0 % |
| 1, 3-Butylene glycol | 5.0 % |
| Ethanol | 12.0 % |
| Sodium alginate | 0.5 % |
| Polyoxyethylene (20) oleyl ether | 0.5 % |
| Perfume | q.s. |
| Antiseptic | q.s. |
| Buffer | q.s. |
| Purified water | Balance |

### Formulation Example 14: Foundation

| | |
|---|---|
| N, N'-di(n-octanoyl)-L-cystine dimethyl ester | 5.0 % |
| Salicylic acid | 0.5 % |
| Liquid paraffin | 10.0 % |
| Polyoxyethylene (20) sorbitan monooleate | 3.5 % |
| Propylene glycol | 3.0 % |
| Titanium oxide | 9.0 % |
| Kaolin | 24.0 % |
| Talc | 42.0 % |
| Coloring pigment | 3.0 % |
| Perfume | q.s. |
| Antiseptic | q.s. |
| Antioxidant | q.s. |

### Formulation Example 15: Liquid hand soap

| | |
|---|---|
| N, N'-diacetyl-L-cystine dimethyl ester | 5.0 % |
| Sodium lauryl sulfate | 30.0 % |
| Betaine | 3.0 % |
| Glycerin fatty acid ester | 1.0 % |
| Phenoxyethanol | 1.0 % |
| EDTA | 0.1% |
| Purified water | Balance |

### Formulation Example 16: Face wash

| | |
|---|---|
| N, N'-di (n-butyryl)-L-cystine diamide | 0.5 % |
| Triethylamine N-lauroylglutamate | 25.0 % |
| Triethanolamine laurate | 5.0 % |
| Polyoxyethylene (4) polyoxypropylene (11) butyl ether | 5.0 % |
| Ethanol | 3.0 % |
| Perfume | q.s. |
| Antiseptic | q.s. |
| Purified water | Balance |

### Formulation Example 17: Shampoo

| | |
|---|---|
| N, N'-dioctanoyl-L-cystine dimethyl ester | 0.1 % |
| Triethanolamine polyoxyethylene (3) lauryl ether sulfate | 3.0 % |
| Sodium polyoxyethlene (3) lauryl ether sulfate | 6.0 % |
| Sodium lauryl salfate | 1.5 % |
| Lauric acid diethanolamide | 3.0 % |
| Lauryl dimethylaminoacetic acid betaine | 2.5 % |
| Cationized cellulose | 0.2 % |
| Ethylene glycol distearate | 2.0 % |
| Perfume | q.s. |
| Antiseptic | q.s. |
| Chelating agent | q. s. |
| Buffer | q. s. |
| Purified water | Balance |

### Formulation Example 18: Hair lotion

| | |
|---|---|
| N, N'-diacetyl-L-cystine dimethyl ester | 1.0 % |
| Lactic acid | 0.02 % |
| Oleyl alcohol | 0.2 % |
| Liquid paraffin | 0.5 % |
| Ethanol | 5.0 % |
| Sorbitol | 4.0 % |
| Polyoxyethylene (20) lauryl ether | 2.5 % |
| Sorbitan monolaurate | 0.5 % |
| Pigment | 0.1% |
| Antiseptic | 0.1 % |
| Perfume | 0.1 % |
| Purified water | Balance |

### Formulation Example 19: Disinfectant detergent

| | |
|---|---|
| N, N'-diacetyl-L-cystine dimethyl ester | 2.0 % |
| Ethanol | 70.0 % |
| Benzalkonium chloride | 1.0 % |
| Chlorhexidine gluconate | 0.5 % |
| 2-Lauryl-N-carboxymethyl-N-hydroxyethylimidazolium betaine | 2.0 % |
| Sodium DL-pyrrolidone carboxylate | 5.0 % |
| N^{α}-cocoylarginine ethyl ester DL-pyrrolidone carboxylate | 1.0 % |
| Purified water | Balance |

### Formulation Example 20: Disinfectant detergent

| | |
|---|---|
| N, N'-dioctanoyl-L-cystine dimethyl ester | 0.1 % |
| Lauryl dimethylaminoacetic acid betaine | 5.0 % |
| Sodium chloride | 1.0 % |
| Benzalkonium chloride | 0.1 % |
| Stearyl trimethyl ammonium chloride | 2.0 % |
| Boric acid | 0.5 % |
| Borax | 0.1 % |
| EDTA | 0.1% |
| Purified water | Balance |

### Formulation Example 21: Bath agent (granule)

| | |
|---|---|
| N, N'-dioctanoyl-L-cystine dimethyl ester | 3.0 % |
| Benzalkonium chloride | 0.1% |
| Sodium sulfate | 44.0 % |
| Sodium bicarbonate | 50.0 % |
| Borax | 0.1 % |
| Sodium carboxymethylcellulose | 1.0 % |
| Pigment | q. s. |
| Perfume | q. s. |

### Industrial Applicability

According to the present invention, there can be provided cosmetics or external preparations for skin which comprises, as the effective ingredient, a chemical peeling agent, a bactericide, an anionic surfactant or a cationic surfactant component in combination with a compound capable of mitigating irritation, inflammation, etc. of the skin caused by these components.

## Claims

1. Skin cosmetics, external preparations for the skin or hair cosmetics which are **characterized by** containing the following components (A) and (B):
Components (A): one or more of members selected from a cystine derivative which may be represented by the following general formula ( l ) and salt thereof 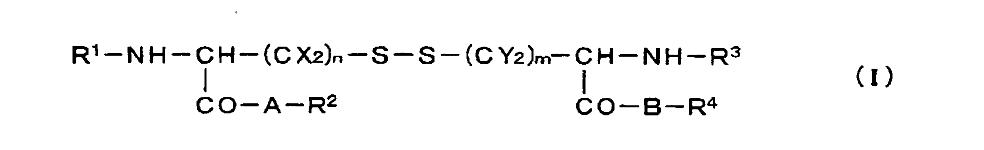 wherein R¹ and R³ represent a hydrogen atom, an aminocarbonyl group, an alkyl group having 1-22 carbon atoms, an acyl group having 2-22 carbon atoms, a hydroxyalkyl group having 1-22 carbon atoms or 3-alkoxy-2-hydroxypropyl group whose alkoxy group has 1-22 carbon atoms, two X and two Y represent each independently a hydrogen or an alkyl group having 1-6 carbon atoms, and n and m represent each independently an integer of 0-5, and A and B represent independently each -O- or -NH-, R² and R⁴ represent a hydrogen atom, an alkyl group having 1-22 carbon atoms, a hydroxyalkyl group having 1-22 carbon atoms or a 3-alkoxy-2-hydroxypropyl group whose alkoxy group has 1-22 carbon atoms.
Components (B): one or more of members selected from a chemical peeling agent, a bactericide and an anionic surfactant

2. The skin cosmetics, external preparations for the skin or hair cosmetics as claimed in claim 1 wherein the cystine derivative is N, N'-diacylcystine dialkyl ester represented by the general formula (II) 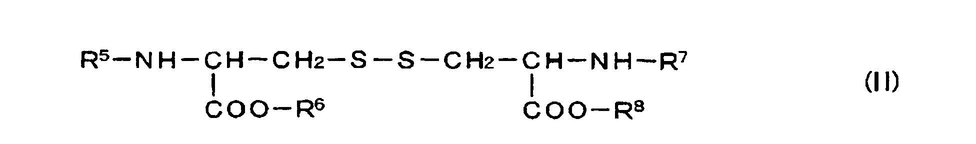 wherein R⁵ and R⁷ represent an acyl group having 2-22 carbon atoms and R⁶ and R⁸ represent an alkyl group having 1-22 carbon atoms.

3. The skin cosmetics, external preparations for the skin or hair cosmetics as claimed in claim 1 wherein the cystine derivative is one or more members selected from N, N'-diacetylcystine dimethyl ester and N, N'-di (n-octanoyl) cystine dimethyl ester.

4. The skin cosmetics, external preparations for the skin or hair cosmetics as claimed in any one of claims 1 to 3 wherein the chemical peeling agent is one or more members selected from a hydroxyl acid, its derivative and salts thereof.

5. The skin cosmetics, external preparations for the skin or hair cosmetics as claimed in claim 4 wherein the hydroxyl acid is one or more members selected from lactic acid and/or glycolic acid their derivatives and salts thereof.

6. The skin cosmetics, external preparations for the skin or hair cosmetics as claimed in any one of claims 1 to 3 wherein the bactericide is one or more members selected from benzalkonium chloride and derivative thereof.

7. The skin cosmetics, external preparations for the skin or hair cosmetics as claimed in any one of claims 1 to 3 wherein the surfactant is one or more members selected from sodium lauryl sulfate and derivative thereof.

8. Skin cosmetics or external preparations for the skin which are **characterized by** containing the following components (C) and (D):
Components (C): one or more of members selected from a cystine derivative which may be represented by the following general formula (I) and salt thereof 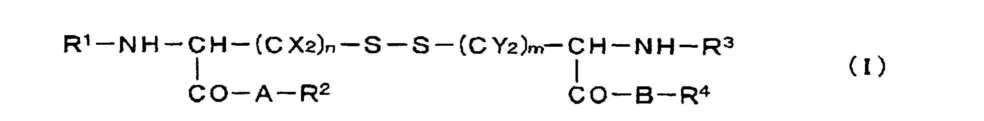 wherein R¹ and R³ represent a hydrogen atom, an aminocarbonyl group, an alkyl group having 1-22 carbon atoms, an acyl group having 2-22 carbon atoms, a hydroxyalkyl group having 1-22 carbon atoms or 3-alkoxy-2-hydroxypropyl group whose alkoxy group has 1-22 carbon atoms, two X and two Y represent each independently a hydrogen or an alkyl group having 1-6 carbon atoms, and n and m represent each independently an integer of 0-5, and A and B represent independently each -O- or -NH-, R² and R⁴ represent a hydrogen atom, an alkyl group having 1-22 carbon atoms, a hydroxyalkyl group having 1-22 carbon atoms or a 3-alkoxy-2-hydroxypropyl group whose alkoxy group has 1-22 carbon atoms.
Components (D): a cationic surfactant.

9. The skin cosmetics, external preparations for the skin or hair cosmetics as claimed in claim 8 wherein the cystine derivative is N, N'-diacylcystine dialkyl ester represented by the general formula (ll) 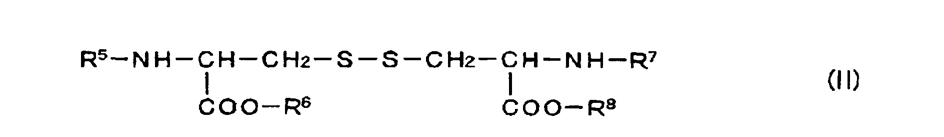 wherein R⁵ and R⁷ represent an acyl group having 2-22 carbon atoms and R⁶ and R⁸ represent an alkyl group having 1-22 carbon atoms.

10. The skin cosmetics or external preparations for the skin as claimed in claim 8 wherein the cystine derivative is one or more members selected from N, N'-diacetyl-L-cystine dimethyl ester and N, N'-di (n-octanoyl) cystine dimethyl ester.

11. The skin cosmetics or external preparations for the skin as claimed in any one of claims 8 to 10 wherein the cationic surfactant is one or more members selected from stearyl trimethyl ammonium chloride and derivative thereof.
